# EUROPEAN PATENT SPECIFICATION

(11) **EP 2 606 357 B1**
(45) Date of publication and mention of the grant of the patent: **05.11.2014**
(21) Application number: 11757919.3
(22) Date of filing: 17.08.2011
(51) Int. Cl.: G01N 33/569

(54) **IDENTIFICATION OF LIGANDS AND THEIR USE**
IDENTIFIZIERUNG VON LIGANDEN UND IHRE VERWENDUNG
IDENTIFICATION DE LIGANDS ET LEUR UTILISATION

(30) Priority: 17.08.2010 GB 201013767
(43) Date of publication of application: 26.06.2013
(73) Proprietor: Isis Innovation Limited, Oxford, Oxfordshire OX2 7SG (GB)
(72) Inventor: OGG, Graham, Oxford Oxfordshire OX3 9DS (GB); HUANG, Li-Chieh, Headington Oxford Oxfordshire OX3 9DU (GB); ASLAM, Aamir, Oxfordshire OX3 9DS (GB)
(74) Representative: Atkinson, Jennifer
(86) International application number: PCT/GB2011/051551
(87) International publication number: WO 2012/022975

(56) References cited:
- WO-A2-2006/009838
- US-A1- 2004 072 262
- US-A1- 2006 228 758
- FRANCISCO BORREGO ET AL: "Structure and function of major histocompatibility complex (MHC) class I specific receptors expressed on human natural killer (NK) cells", MOLECULAR IMMUNOLOGY, vol. 38, no. 9, 1 February 2002 (2002-02-01), pages 637-660, XP55015347, ISSN: 0161-5890, DOI: 10.1016/S0161-5890(01)00107-9
- NATHALIE T. JONCKER ET AL: "Regulation of NK cell responsiveness to achieve self-tolerance and maximal responses to diseased target cells", IMMUNOLOGICAL REVIEWS, vol. 224, no. 1, 1 August 2008 (2008-08-01), pages 85-97, XP55015344, ISSN: 0105-2896, DOI: 10.1111/j.1600-065X.2008.00658.x
- ANDREAS MACKENSEN ET AL: "Phase I study in melanoma patients of a vaccine with peptide-pulsed dendritic cells generatedin vitro from CD34+ hematopoietic progenitor cells", INTERNATIONAL JOURNAL OF CANCER, vol. 86, no. 3, 1 May 2000 (2000-05-01), pages 385-392, XP55015232, ISSN: 0020-7136, DOI: 10.1002/(SICI)1097-0215(20000501)86:3<385: :AID-IJC13>3.0.CO;2-T
- STONE D J ET AL: "HLA-restricted epitope identification and detection of functional Tcell responses by using MHC-peptide and costimulatory microarrays", PROCEEDINGS OF THE NATIONAL ACADEMY OF SCIENCES OF USA, NATIONAL ACADEMY OF SCIENCE, WASHINGTON, DC; US, vol. 102, no. 10, 8 March 2005 (2005-03-08), pages 3744-3749, XP002383140, ISSN: 0027-8424, DOI: 10.1073/PNAS.0407019102
- SOEN Y ET AL: "DETECTION AND CHARACTERIZATION OF CELLULAR IMMUNE RESPONSES USING PEPTIDE-MHC MICROARRAYS", PLOS BIOLOGY, PUBLIC LIBRARY OF SCIENCE, US, vol. 1, no. 3, 1 December 2003 (2003-12-01), pages 429-438, XP009044714, ISSN: 1544-9173, DOI: 10.1371/JOURNAL.PBIO.0000065
- RUI GAN ET AL: "Microbeads display of proteins using emulsion PCR and cell-free protein synthesis", BIOTECHNOLOGY PROGRESS, vol. 24, no. 5, 1 September 2008 (2008-09-01), pages 1107-1114, XP55014985, ISSN: 8756-7938, DOI: 10.1002/btpr.43
- KFIR OVED ET AL: "Antibody-mediated targeting of human single-chain class I MHC with covalently linked peptides induces efficient killing of tumor cells by tumor or viral-specific cytotoxic T lymphocytes", CANCER IMMUNOLOGY, IMMUNOTHERAPY, SPRINGER, BERLIN, DE, vol. 54, no. 9, 1 September 2005 (2005-09-01), pages 867-879, XP019333169, ISSN: 1432-0851, DOI: 10.1007/S00262-005-0666-5

## Description

This invention relates to the identification of ligands, and in particular to the identification of peptide ligands for T-cell receptors and/or NK-cell receptors and/or NKT-cell receptors. The invention also relates to carriers for use in the identification of ligands, and to a kit for the identification of ligands, and to the use of identified ligands.

With the continued need for drug discovery and the continued quest to understand disease and infection, there remains a need for improved methods to study protein:protein interactions and to identify potential protein ligands for receptors. There are currently numerous methods available to study protein interactions and to screen for potential ligands, these include, but are not limited to, affinity chromatography, phage display, the yeast two hybrid system, protein microarrays and 3D structural analysis using X-ray crystallography and/or NMR.

Ideally, the mechanism of probing protein interactions or probing for potential ligands will directly link any identified protein or peptide to its encoding DNA. Examples of methods which allow this include the yeast two hybrid and phage display systems. However, these both have the disadvantage that in addition to the proteins under study there are other proteins, that is on both the phage and/or the yeast, which can interfere with binding or actually bind themselves. For example, this can lead to non-specific interactions which can be difficult to distinguish from interactions of interest. Furthermore these methods commonly use fusion proteins which can alter the conformation of "the hunter" or "the bait" and influence binding. The produced proteins may be toxic to the yeast or the phage or influence their replication. Post-translational modifications may also be important for binding between the natural ligands and these may not be present in the displayed proteins using yeast or phage based systems.

Stone, J. et al. PNAS (2005), 102(10):3744-3749 discloses MHC class I or II peptide complexes arrayed to a solid support and their use to identify T cell epitopes, wherein T cells bind via their TCRs specifically to the MHC-peptide complexes they recognise to the said array.

The advantage of linking the peptide to the encoding DNA is that methods available to sequence the DNA are far more sophisticated than those available to sequence protein. In particular, DNA sequencing is much more rapid and is cheaper than protein sequencing. DNA sequencing can be successful on very small samples - small in both length and molar amounts. Furthermore, DNA samples can be easily amplified to provide more DNA if needed. DNA sequencing can be undertaken by traditional Sanger based methodology or by various high throughput sequencing approaches (Sequencing technologies - the next generation. Metzker ML. Nat Rev Genet. 2010 Jan;11(1):31-46). In contrast, protein sequencing can be via Edman degradation or through mass spectrometry approaches (Hanno Steen & Matthias Mann. The abc's (and xyz's) of peptide sequencing. Nature Reviews Molecular Cell Biology, 5:699-711, 2004).

An aim of the present invention is provide an alternative system to screen for ligands, in particular to screen for ligands for cell surface receptors. In a preferred embodiment the invention will provide an alternative system to screen for ligands for T-cell receptors and/or NK-cell receptors and/or NKT-cell receptors. None of the known methods which consider protein interactions lend themselves to screening for epitopes for T-cell receptors and/or NK-cell receptors and/or NKT-cell receptors which requires the epitope to be correctly presented in order for the T-cell and/or NK-cell and/or NKT-cell to recognise it.

In order for a T-cell receptor and/or an NK-cell receptor and/or an NKT-cell receptor to recognise an epitope is must be correctly presented on an MHC (major histocompatibility complex) molecule or an MHC-like molecule. For example, when a foreign pathogen enters the body, specific cells called antigen-presenting cells (APCs) engulf the pathogen through a process called phagocytosis. Proteins from the pathogen are then digested into small pieces (peptides) and loaded onto MHC molecules (particularly Human leukocyte antigen (HLA) class II, also known as MHC class II). In humans MHC antigens/molecules may also be referred to as HLA antigens/molecules, and the terms MHC and HLA are used interchangeably herein. The peptides on the HLA molecules are then displayed by the antigen presenting cells for recognition by CD4 + T cells via T-cell receptors. T-cells which bind to the foreign pathogen epitope via a T cell receptor then produce a variety of immune responses in response to the pathogen. Native host proteins may also be presented in this way.

Through a similar process, proteins (both native and foreign, such as the proteins of viruses) produced inside most cells are displayed on MHC molecules (particularly MHC class I) on the cell surface. Again T-cell receptors can then bind to the MHC molecule allowing infected cells to be recognized and destroyed by components of the immune system (specifically CD8 + T cells) stimulated in response to the foreign peptides.

In addition to T-cell receptors, MHC molecules and MHC-like molecules may also bind to receptors on NK cells (natural killer cells) and NK-T cells. This binding may influence cell function.

Natural killer (NK) cells are lymphocytes that are part of the innate immune system. They are an important part of the first line of defence that protects the body from pathogen invasion and malignant transformation.

NKT cells are a subset of T cells that co-express a T cell receptor (TCR), but also express a variety of molecular markers that are typically associated with NK cells, such as NK1.1. Although NKT cells are thought to primarily recognise lipids presented by CD1 molecules, it is possible that other types of NKT cells are able to recognise peptides presented by MHC or MHC-like molecules.

The MHC proteins which form the MHC molecules or antigens are unique to individuals, and are used by the immune system to differentiate self cells and non-self cells. More specifically, cells displaying a person's own MHC molecules presenting normal host peptides are identified by T-cells from that person as self and in normal, non disease states, do not initiate an immune response.

Detecting the specificity of T-cell receptors or NK-cell receptors or NKT-cell receptors at a disease site will generate insights into disease pathogenesis. For example, the identification of an epitope that a T-cell receptor recognises may allow the proteins/genes involved in a disease state to be identified and studied further. Furthermore, by identifying what epitope a T-cell receptor and/or an NK-cell receptor and/or an NKT-cell receptor recognises will allow the identification of the disease relevant part of a known protein. Ultimately this may lead to the identification of potential new therapeutic, diagnostic or prevention targets. It may be of interest to determine the specificity of T cell receptors and/or NK-cell receptors and/or NKT-cell receptors within diseased tissues, which may include cancerous tissue, tissue from an individual with an autoimmune disease or with an allergy, infected tissue or diseased tissue of completely unknown pathogenesis. Comparisons may be made between the diseased tissue and adjacent or non-adjacent normal tissue.

According to a first aspect, the invention provides a method to identify a peptide and/or its encoding DNA, wherein the peptide binds to a T-cell receptor or an NK-cell receptor or an NKT-cell receptor, the method comprising: providing a carrier to which is attached a peptide and its encoding DNA; providing an MHC and/or an MHC-like molecule; and exposing the peptide to a T-cell receptor and/or an NK-cell receptor and/or an NKT-cell receptor in the presence of the MHC or MHC-like molecule. Preferably the method of the invention also includes providing β2 microglobulin togther with the MHC and/or the MHC-like molecule, and exposing the peptide to a receptor in the presence of β2 microglobulin and the MHC or the MHC-like molecule.

The configuration adopted on the carrier by the peptide, the β2 microglobulin and/or the MHC or the MHC-like molecule preferably means that the peptide is presented in such a manner that it will be recognised by a T-cell receptor and/or an NK-cell receptor and/or an NKT-cell receptor.

Preferably both β2 microglobulin and/an MHC or MHC-like molecule are used.

In a preferred embodiment, the invention provides a method to identify a peptide and/or its encoding DNA, wherein the peptide binds to a T-cell receptor or an NK-cell receptor or an NKT-cell receptor, the method comprising: providing a carrier to which is attached a peptide and its encoding DNA; providing β2 microglobulin and MHC and/or an MHC-like molecule; and exposing the peptide to a T-cell receptor and/or an NK-cell receptor and/or an NKT-cell receptor in the presence of the β2 microglobulin and the MHC or MHC-like molecule.

Preferably the method of the invention relates to T-cell receptors.

Preferably the method of the invention is performed *in vitro.*

By identifying an epitope/peptide that is able to bind to a T-cell receptor, and/or an NK-cell receptor and/or an NKT-cell receptor the DNA which encodes the epitope/peptide recognised by the T-cell receptor and/or an NK-cell receptor and/or an NKT-cell receptor can be readily recovered, amplified and sequenced. From the sequence of the DNA the protein from which the peptide is derived may be deduced.

The MHC or MHC-like molecule may be the full length naturally occurring MHC or MHC-like molecule, or it may be a functional variant thereof. The functional variant may be any modified variant or truncation or fusion of an MHC or MHC-like molecule. The molecule is considered functional if it can fold correctly to allow the binding of conformationally dependent antibodies (eg W6/32 for HLA class I) or presentation of a peptide to a T-cell receptor, and/or an NK-cell receptor and/or an NKT-cell receptor (Choi et al J Immunol Methods. 2002 Oct 1;268:35-41).

Various functional variants of MHC molecules have been used as functional MHC molecules. For example human alphal and alpha2 regions have been fused to murine alpha3 regions of MHC molecules to present antigen to T cells (Choi et al J Immunol Methods. 2002 Oct 1;268:35-41). A further example is modification of the MHC sequence to alter CD8 binding (Wooldridge et al. Eur J Immunol. 2007 May;37:1323-33). These and other modified forms of MHC or MHC-like molecules may be used in the invention.

A T-cell receptor is a molecule found naturally on the surface of T-cells which recognises antigens/peptide epitopes presented on an MHC molecule or an MHC-like molecule. The T-cell receptor may be composed of alpha-beta chains or gamma-delta chains. Preferably, when a T-cell receptor binds to an antigen/peptide epitope *in vivo* it signals a biochemical cascade reaction which results in an immune response.

NK receptors may include, but not be limited to, NKG2 family members, conserved T cell receptor chains, CD94, leukocyte Ig-like receptor family, killer inhibitory receptor, Ly49 family, NKp30, 46, NKp44, NKp80, CD244 (2B4), KLRG1, NKR-P1, DNAM-1, PILR.

NK-T cells have variable or invariant T cell receptors that bind to CD1 family members that present lipid structures. These are likely to be important in many aspects of immune responses including pathogen-specific and auto-reactive immunity.

Ligands for the NK or NK-T receptors are diverse and include, but are not limited to, MIC family molecules, HLA-E, HLA-G, CD1a, CD1b, CD1c, CDld, BAT-3, HSPG, B7-H6, HSPG, AICL, UL18, CD48, Cadherins, PVR, CD122 and ULBP family molecules in humans, as well as CD1, RAE-1, MULT-1, H2-Q (such as Qa-1), H2-T (such as H2-T10, H2-T22), H2-M (such as H2-M3), CD1d, Rae-1, UL18, Ocil/Clr-b, CD48, Cadherins, PVR, CD122, CD99, m157 and H-60 molecules in the mouse. Other non-classical HLA molecules eg HLA-D, F, H are also important; as are other pathogen-expressed proteins (eg viral HA).

The method of the invention has the advantage that it is very simple. Preferably it also has the advantage that it is free from interference by unwanted proteins, this is in contrast to phage display and the yeast two hybrid system where host proteins can interfere with the binding of the expressed peptide. Preferably the peptide, the MHC or MHC-like molecule and the β2 microglobulin are the only proteins present on the carrier in the subject invention.

A further advantage of the invention is that the carrier may be multivalent, that is it may carry multiple copies of each peptide and its encoding DNA. This will increase the chance of interaction and also improve the rate of recovery.

Biological *in vivo* based systems (eg yeast or phage) can be limited in terms of library size because of limitations in handling large numbers of biological particles and efficient take up of library components. In contrast the *in vitro* system of the invention is not dependent on expression by yeast or phage and therefore can offer greater library sizes.

The method of the invention may further include the step of recovering the peptide, and/or its encoding DNA, which bound to a T-cell receptor and/or NK-cell receptor and/or NKT-cell receptor. In a preferred embodiment the DNA encoding the peptide which bound to the T-cell receptor and/or the NK-cell receptor and/or the NKT-cell receptor is recovered.

Preferably the peptide used is between about 4 and about 20 amino acids, preferably the peptide is between about 7 and about 15 amino acids, preferably the peptide is between about 7 and about 12 amino acids, preferably the peptide is between about 8 and about 12 amino acids, preferably the peptide is between about 8 and about 10 amino acids, preferably the peptide is about 9 amino acids, preferably the peptide is 9 amino acids.

The peptide may be randomly generated or derived from a source library, for example, from a particular human or non-human cell type.

If the peptide is recovered it may be sequenced. The sequence may then be analysed to determine which protein the peptide is derived from, typically this is achieved using sequence databases.

If the nucleic acid encoding the peptide is recovered it may be sequenced. The sequence may than be analysed to determine the peptide it encodes and/or to determine the gene from which it is derived and/or to determine the protein from which the peptide it encodes is derived. The nucleic acid sequence may be determined through conventional Sanger based methodology or by using high throughput screening approaches. The advantage of high throughput screening approaches is that large numbers of sequences can be rapidly sequenced.

Having obtained the sequence, either of the DNA encoding the peptide or the peptide itself, of one or more of the peptides that bind to a T-cell receptor and/or an NK-cell receptor and/or an NKT-cell receptor, the sequences can then be compared to protein and DNA sequence databases to identify possible proteins or genes from which the peptide epitope is derived. Preferably the method will allow multiple different epitopes to be identified which together will allow the protein from which they are derived to be identified.

To improve the analysis the T-cells and/or the NK-cells and/or the NKT-cells may first be probed with a carrier carrying peptide with a fixed epitope sequence before probing with an epitope library. Bioinformatics could then be used to calculate a cut-off (based on the fixed epitope sequence) above which non-fixed sequences may be relevant. The identified sequences could then be applied to database searches to examine common patterns that emerge. This system may be amenable to subsequent rounds of selection to enrich for sequences of interest. Further approaches to enhance analytic capacity would be to compare the specificity of T-cells or NK-cells or NKT-cells within lesional tissue compared to non-lesional tissue. In addition the sequences generated in one round of selection can be used to derive a refined library for subsequent rounds of selection.

The β2 microglobulin and/or the MHC or MHC-like molecule may be included to allow the peptide to be presented in the correct configuration such that it may be recognised by a T-cell receptor and/or an NK-cell receptor and/or an NKT-cell receptor.

As discussed previously, MHC molecules may also be referred to in humans as HLA molecules. HLA refers to a group of proteins referred to as human leukocyte antigens (HLA). Any one of the HLA or HLA-like molecules may be used in the invention, as can any analogous MHC or MHC-like molecules from mice or other species. The different classes of HLAs include, but are not limited to, HLA class I proteins (A, B & C) which largely present peptides from inside the cell (including viral peptides if present); HLA class II proteins (DP, DM, DOA, DOB, DQ & DR) which largely present antigens from outside of the cell to T-lymphocytes; and HLA class III proteins which form part of the complement system. Preferably in the method of the invention the HLA is a class I or a class II HLA. More preferably when the structure is refolded with beta-2-microglobulin the HLA is a class I HLA. The analogous molecules in mice include H-2 family members and I-A and I-E family members and in the rat include RT1 family members.

HLA-like or MHC-like molecules include ligands for T cell receptors or NK receptors or NK-T cell receptors as detailed above.

β2 microglobulin is also known as B2M, and is present on virtually all nucleated cells. In humans, the β2 microglobulin protein is encoded by the B2M gene.

The MHC molecules and MHC-like molecules and β2 microglobulin work together in nature to present peptides to the body's immune system, and in particular to the T-cell receptors, and also to other receptors such as NK-cell receptors or NKT-cell receptors.

Preferably in the method of the invention the MHC or MHC-like molecule and β2 microglobulin proteins interact with the peptide attached to the carrier to put the peptide into a confirmation such that it can be recognised by a T-cell receptor and/or an NK-cell receptor and/or an NKT-cell receptor. MHC-like molecules for use in the invention are molecules that have an MHC like three-dimensional structure and are able to present a peptide to a T-cell, NK-cell or NKT-cell, The MHC-like molecule may not associate with beta-2-microglobulin eg MIC-A.

The β2 microglobulin may be added exogenously to the peptide. Alternatively the β2 microglobulin may be provided connected to the peptide via a peptide linker, preferably via a flexible peptide linker.

Similarly the MHC or MHC-like molecule may be added exogenously to the peptide. Alternatively the MHC or MHC-like molecule may be provided connected to the peptide and/or the β2 microglobulin via a peptide linker, preferably via a flexible peptide linker.

In one embodiment both the MHC or MHC-like molecule and the β2 microglobulin are provided linked to the peptide. In another embodiment both the MHC or MHC-like molecule and the β2 microglobulin are provided exogenously. In a further embodiment the MHC or MHC-like molecule is provided linked to the peptide and the β2 microglobulin is added exogenously. In a further embodiment the β2 microglobulin is provided linked to the peptide and the MHC or MHC-like molecule is added exogenously.

When the MHC or MHC-like molecule and/or the β2 microglobulin are linked to the peptide, they are preferably linked by a flexible peptide linker. A flexible peptide linker is series of amino acids which connects two defined regions, such as the peptide and the β2 microglobulin, and allows the two defined regions to move. Preferably the linker allows the two regions to have locational freedom. Preferably the linker allows the regions it links to form their preferred configuration whilst still being linked.

The carrier may be a solid support. The solid support may be a column, a plate surface (such as the surface of a tissue culture plate or the surface of a multiwell plate), a bead or any other suitable support.

If the carrier is a bead, the bead may be of any polymeric material, such as polystyrene, although non-polymeric materials, such as silica, may also be used. Other materials which may be used include styrene copolymers, methyl methacrylate, functionalised polystyrene, glass, silicon, and carboxylate. Optionally the beads may be magnetic, which facilitates their isolation after being used in reactions.

Preferably the beads are microspheres with a diameter from about 0.1 to about 10 microns. The beads may alternatively be any small discrete particle they need not be spherical in shape. Preferably they will be similar in size to a sphere of about 0.1 to about 10 microns, but different sized structures including nanometre sized particles may also be used.

In a preferred embodiment the carrier is a bead. Preferably each bead carries multiple copies of the same peptide and the same DNA encoding the peptide. The number of copies may range from about 2 to a million or more. Preferably there are at least 10 copies, at least 20 copies, at least 50 copies, at least 100 copies, at least 500 copies, at least 1000 copies, at least 10,000 copies, at least 100,000 copies. Preferably only one peptide species and only one DNA species is found on each bead. The method of the invention may use multiple beads wherein different beads have different peptides and DNA sequences attached.

In a preferred embodiment, *in vitro* transcription/translation (IVTT) is used to produce the peptide, and if applicable the β2 microglobulin and/or the MHC or MHC-like molecule, which is bound to the carrier surface. The β2 microglobulin and/or the MHC or MHC-like molecule may also be produced by IVTT. The β2 microglobulin and/or MHC or MHC-like molecule may be attached to the peptide via a flexible peptide linker also produced by IVTT. The peptide, β2 microglobulin and/or MHC or MHC-like molecule may be synthesised by IVTT as a single linked product.

If IVTT is used, it may be emulsion IVTT (Directed Evolution of Proteins In Vitro Using Compartmentalization in Emulsions Davidson, Dlugosz, Levy, Ellington, Current Protocols in Molecular Biology 24.6.1-24.6.12, July 2009)

The peptide, and if applicable the β2 microglobulin and/or the MHC or MHC-like molecule, may be synthesised from DNA attached to the carrier. Preferably this is achieved using IVTT. The peptide, and if applicable the β2 microglobulin and/or the MHC or MHC-like molecule, may then be attached to the carrier. Alternatively, the peptide, and if applicable the β2 microglobulin and/or the MHC or MHC-like molecule, could be produced remote from the carrier and then attached to the carrier. The attachment may be covalent or non-covalent.

If produced by IVTT the peptide construct may also comprise a tag which attaches the peptide to the carrier. The tag may be a steptavidin binding protein, which when used in combination with a streptavidin coated/treated carrier attaches the peptide construct to the carrier. Alternatively a his-tag or HA-tag may be used. The skilled man will appreciate that these are merely examples and that other tags may be used.

Reference herein to a peptide construct is intended to refer to the product of the translation of a DNA molecule attached to the carrier, the construct may comprise the peptide and one or more of an MHC or MHC-like molecule, β2 microglobulin, a tag and a linker.

The peptide construct may be attached to the carrier following emulsion PCR and emulsion IVTT. In this embodiment the DNA encoding the peptide and any other necessary components, for example one or more of a tag, β2 microglobulin, an MHC or MHC-like molecule, a linker, a promoter and a terminator, is first attached to the carrier by PCR. Preferably a primer for the DNA encoding the peptide and any other necessary components is first attached to the carrier, and PCR is then used to amplify the template DNA encoding the peptide and any other necessary components using the primer attached to the carrier. The DNA encoding the peptide may be randomly generated or derived from a source library for example a peptide human or non-human cell type. The primer may be attached to the carrier by any means known in the art. It may be bound covalently or non-covalently. DNA amplified by the PCR may be captured on the carrier. This may be achieved by using a tag on one of the primers, for example, the tag may be a biotin moiety and which would allow the amplified DNA to be captured on a streptavidin carrier. The primer carrying the tag may be the primer which is not bound to the carrier prior to PCR.

Preferably the PCR and/or the IVTT is carried out in an emulsion (as described in Directed Evolution of Proteins In Vitro Using Compartmentalization in Emulsions Davidson, Dlugosz, Levy, Ellington, Current Protocols in Molecular Biology 24.6.1-24.6.12, July 2009; Miniaturizing chemistry and biology in microdroplets. Kelly BT, Baret JC, Taly V, Griffiths AD. Chem Commun 2007 May 14;(18):1773-88). The emulsions may be made by stirring or agitating an oil and aqueous mixture to form small droplets of water in the oil. The emulsion may be stabilised by including a surfactant. Preferably where emulsion PCR and/or emulsion IVTT is carried out the carrier is a bead. Preferably each droplet in the emulsion contains only one bead. Preferably the aqueous phase of the emulsion carries all the reagents and enzymes necessary to carry out the PCR or the IVTT. For example, for a PCR reaction the aqueous phase preferably contains a DNA polymerase and nucleotides.

In order to move from an emulsion PCR reaction to an emulsion IVTT reaction the emulsion involved in the PCR reaction must be broken, the carrier (preferably beads) recovered, and then a new emulsion made with the carrier, IVTT reagents and enzymes. Emulsions can be broken or disrupted by any means known in the art. One well known method is to add more detergent/surfactant.

If emulsion IVTT is performed and the carrier is a bead, preferably each bead has multiple copies of the encoding DNA attached to the bead. Preferably an individual bead has DNA of only one sequence attached. Preferably after IVTT each bead has multiple copies of the same DNA and the same peptide/protein product encoded by the DNA.

The method may include the step of folding the peptide, β2 microglobulin and MHC or MHC-like molecule such that they are in a configuration that would be recognised by a T-cell receptor and/or an NK-cell receptor and/or an NKT-cell receptor.

If the carrier is a bead the beads bound to a receptor may be readily recovered by removing any unbound beads using size based exclusion or magnetic or fluorescence based cell sorting

The T-cell receptors and/or the NK-cell receptors and/or the NKT-cell receptors used in the method of the invention may be provided as isolated proteins/receptors, and/or in membrane fragments, and/or on cells in mono culture, and/or in a mixed culture of cells, and/or in a mixed population of cells which includes T-cells and/or NK cells and/or NKT-cells, such as found in a tissue sample.

The method of the invention may be used to identify peptide epitopes important in pathogen recognition or disease. The method of the invention may be used to identify peptide epitopes important in autoimmune conditions, malignant conditions, infection and/or in allergy.

The method of the invention may be used to identify potential epitopes for T-cell receptors on T-cells and/or NK-cell receptors on NK-cells and/or NKT-cell receptors on NKT-cells contained within a tissue sample. The tissue sample may be a sample of diseased tissue, such as tumour tissue or bacterially infected tissue, and the potential epitopes may be identified by exposing a carrier displaying a library of peptides to cells from the tissue sample and isolating any peptides, or the DNA encoding any peptides, that bind to cells in the tissue sample.

According to another aspect, the invention provides a method to identify a DNA encoding a peptide which binds to a T-cell receptor and/or an NK-cell receptor and/or an NKT-cell receptor, the method comprising providing a bead carrier to which is attached a peptide and the DNA encoding the peptide, exposing the peptide in the presence of β2 microglobulin and MHC or MHC-like molecule to a T-cell receptor and/or an NK-cell receptor and/or an NKT-cell receptor, recovering any beads that bound to or were internalised by the T-cell receptor and/or the NK-cell receptor and/or the NK-cell receptor.

In a preferred embodiment, cells expressing a receptor that recognise a peptide on the bead carrier internalise the carrier and peptide.

The method may further comprise the step of recovering the DNA from the recovered beads and sequencing the DNA.

Preferably the peptide and β2 microglobulin are provided connected by a flexible peptide linker, such that both are attached to the bead.

The T cells and/or NK cells and/or NKT cells expressing the T-cell receptor or NK-cell receptor or NKT-cell receptor may be provided in a tissue sample, the tissue sample may have been homogenised to allow access to the component cells. The tissue sample may be a sample of normal or diseased or infected tissue.

Preferably each bead has multiple copies of the peptide and the DNA encoding the peptide attached. Preferably at least 10 copies, preferably at least 100 copies, preferably at least 1000 copies,

According to a further aspect the disclosure provides the use of epitopes, and/or the proteins from which they are derived, identified by the method of the invention, as a target for diagnostic, prognostic, therapeutic or preventative agents.

According to another aspect, the invention provides a carrier to which is attached a peptide, DNA encoding the peptide, and β2 microglobulin and/or an MHC or MHC-like molecule. The β2 microglobulin and/or MHC or MHC-like molecule may be attached to the carrier as part of a larger construct which includes the peptide. The β2 microglobulin and/or MHC or MHC-like molecule may be connected to the peptide via a peptide linker, preferably a flexible peptide linker.

Preferably the peptide is connected to β2 microglobulin via a peptide linker, and the MHC or MHC-like molecule is connected either to the peptide and/or the β2 microglobulin via a further peptide linker. Preferably the linker is a flexible peptide linker.

Preferably the peptide is connected to an MHC or MHC-like molecule via a peptide linker, and the β2 microglobulin is connected to either the peptide or the MHC or MHC-like molecule via a further a linker. Preferably the linker is a flexible peptide linker.

The carrier preferably has on its surface a peptide, an MHC or MHC-like molecule and/or β2 microglobulin configured in such a manner that the peptide would be recognised by a T-cell receptor and/or an NK-cell receptor and/or an NKT-cell receptor. The β2 microglobulin and/or MHC or MHC-like molecule may be attached to the carrier, either directly or via the peptide or via each other, or they may be located at the carrier surface but not physically attached.

Preferably multiple copies of the peptide and encoding DNA are attached to the bead, preferably at least 10, 100, 1000 or more copies are attached,

According to yet another aspect, the invention provides the use of a carrier according to the invention to identify an epitope recognised by a T-cell receptor and/or an NK-cell receptor and/or an NKT-cell receptor.

According to a further aspect the invention provides a kit for screening for a T-cell epitope and/or an NK-cell epitope and/or an NKT-cell epitope, wherein the kit comprises a carrier to which is attached a peptide, DNA encoding the peptide, and β2 microglobulin and/or an MHC or MHC-like molecule. The kit may also include instructions to fold the peptide, β2 microglobulin and/or MHC or MHC-like molecule such that the peptide is in the correct configuration to be recognised by a T-cell receptor and/or an NK-cell receptor and/or an NKT-cell receptor. The kit may also include instructions to expose the carrier to T-cell receptors and/or NK-cell receptors and/or NKT-cell receptors and to isolate peptides, or the DNA encoding the peptides, that bind to the T-cell receptors and/or NK-cell receptors and/or NKT-cell receptors.

The β2 microglobulin and/or the MHC or MHC-like molecule may be provided attached to the carrier, or they may be located at or near the carrier surface.

According to another aspect, the invention provides a method to identify a nucleotide sequence encoding a peptide that binds to a T-cell receptor and/or an NK-cell receptor and/or an NKT-cell receptor comprising amplifying and sequencing the DNA attached to carrier carrying a peptide which binds to a T-cell receptor or NK-cell receptor or NKT-cell receptor.

The skilled person will appreciate that all preferred feature of the invention described with reference to only some aspects of the invention can be applied to all aspects of the invention.

Preferred embodiments of the present invention will now be described, merely by way of example, with reference to the following drawings and examples.

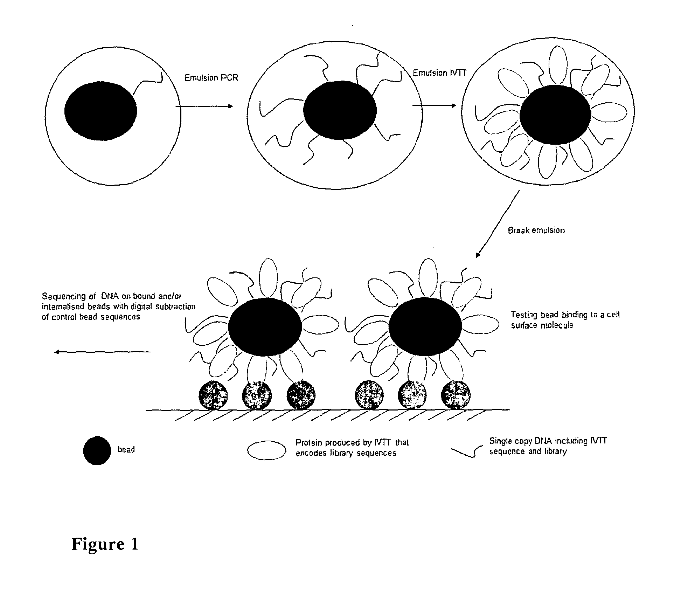

**Figure 1** - is a schematic illustration of emulsion PCR and emulsion IVTT using beads, the beads and bound DNA and peptide are then shown being passed over cell surface molecules in a screen for epitopes are recognised the cell surface molecules;

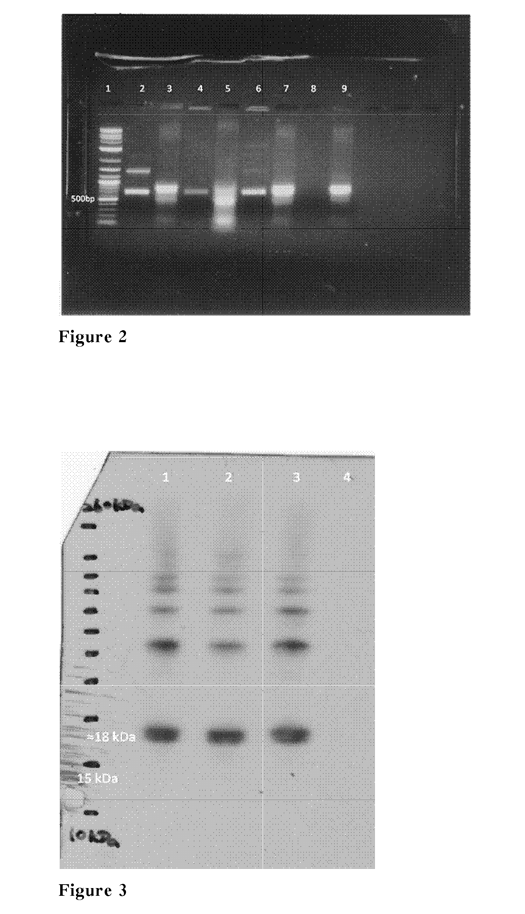

**Figure 2** - shows XbaI restriction digestion of post IVTT bead-DNA-protein complexes. Lane 1 - 2-log DNA ladder; Lane 2 - 5'-bio-for ward primer attached to the beads; Lane 3 - supernatant of (2) after magnet separation; Lane 4 - 5'-bio-reverse primer attached to the beads; Lane 5 - supernatant of (4); Lane 6 - 5'-bio-for and 5'-bio-rev; Lane 7 - supernatant of (6); Lane 8 - negative control: 5'-bio-forward primer attached to the beads, no DNA template; Lane 9 - supernatant of (8);
**Figure 3** **-** shows the Western blot result of human beta-2-microglobulin staining on emulsion-bead IVTT. Lane 1 - shows emulsion IVTT with 5'-bio-forward primer-bead-protein, DNA template: BZLF1 construct. Lane 2 - shows emulsion IVTT with 5'-bio-reverse primer-bead-protein DNA template: BZLF1 construct; Lane 3 - shows emulsion IVTT with 5'-bio-forward and reverse primer-bead-protein DNA template: BZLF1 construct; Lane 4 shows emulsion IVTT negative control, no DNA template added.

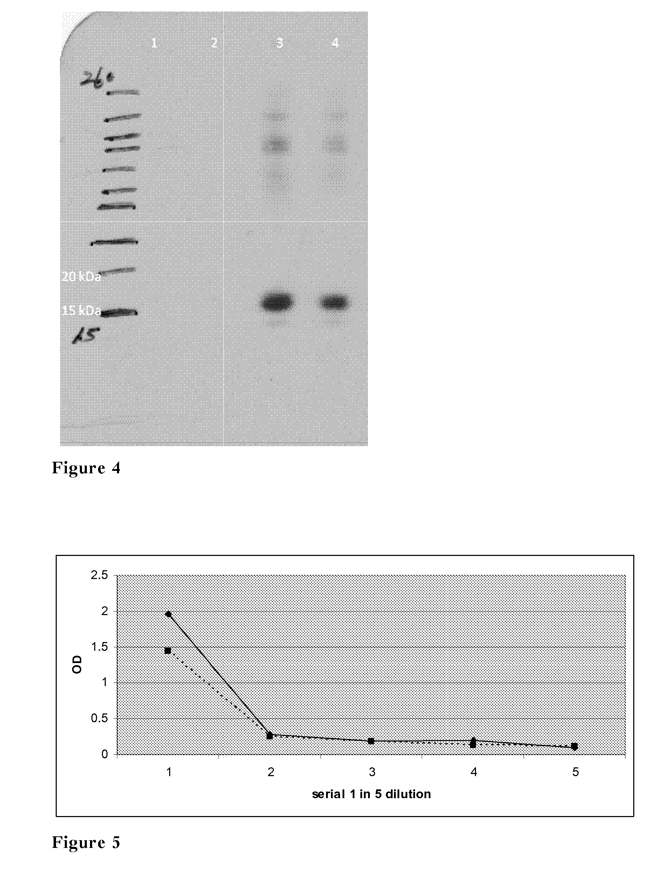

**Figure 4** **-** shows Western blot result of hemagglutinin tag staining on emulsion-bead IVTT. Lane 1 - shows Emulsion IVTT with 5'-bio-forward primer-bead-protein DNA template: BZLF1 construct; Lane 2 - shows Emulsion IVTT with 5'-bio-reverse primer-bead-protein DNA template: BZLF1 construct; Lane 3 - shows Emulsion IVTT with 5'-bio-forward primer-bead-protein DNA template: randomised epitope construct; Lane 4 - shows Emulsion IVTT with 5'-bio-reverse primer-bead-protein DNA template: randomised epitope construct.
**Figure 5** **-** illustrates that a peptide, MHC and β2 microglobulin can be correctly folded on a carrier bead. The solid lines show the ELISA optical density (OD) for a random peptide, MHC and β2 microglobulin refolded on a bead, and the dotted lines show the OD for the a fixed BZLF1 peptide, MHC and β2 microglobulin refolded on a bead.

### Coupled PCR and in vitro transcription/translation reaction in a bead emulsion

A DNA template was designed containing the start and stop sequences for in vitro transcription translation (IVTT) surrounding the sequence of human beta-2-microglobulin, a linker and a fixed or random stretch of amino acids. Different approaches to couple the generated protein back to the beads including using a streptavidin binding peptide and using an HA tag were considered. The sequences are described below.

### Materials

The following materials were used in the experiments described herein: Dynabeads M270 streptavidin (Invitrogen); mineral oil (Sigma); Span-80 (Sigma); Tween-80 (Sigma); Triton X-100 (Sigma); RTS 100 E. coli cell-free transcription/translation high yield system (Roche); expand high fidelity PCR system; dNTP pack (Roche); tris-buffered saline (Invitrogen); H₂O saturated diethyl ether (Sigma); Restriction endonuclease XbaI (New England Biolabs); Antibodies: mouse monoclonal anti-human beta-2-microglobulin (BBM1) (Abcam), Goat polyclonal anti-mouse conjugated Horseradish Peroxidase (Dako), Rat monoclonal anti-hemagglutinin conjugated Horseradish Peroxidase (Roche); Hybond-C extra nitrocellulose membrane (GE healthcare); NuPAGE protein system (Invitrogen); NuPAGE 12% Bis-Tris Midi gel; NuPAGE MES SDS running buffer; NuPAGE transfer buffer PBST (PBS Tween 20 1:1000); ECL developing reagent (GE healthcare); X-ray film 130mmx180mm (Fujifilm).

### DNA sequences

EBV lytic cycle protein BZLF1 was used as a positive control in a DNA template for coupled emulsion PCT and IVTT. The DNA template was as follows:

| **T7 promoter** | **BZLF1** | **Flexible linker** | **Human β2m** | **linker** | **Streptavidin binding protein** | **linker** | **T4 terminator** |
|---|---|---|---|---|---|---|---|
| gatctcgatcccgcgaaattaatacgactcactatagggagaccacaacggtttccctctag S R S R E I N T T R Y R E T T T V S L - aaataattttgtttaactttaagaaggagatataccatgcgagcaaaatttaaacaactg K - F C L T L R R R Y T M R A K F K Q L ttaggcggtggtggcggagggtctggtgggtctggcggtagtggcggcattcagcgcact L G G G G G G S G G S G G S G G I Q R T ccgaaaatccaggtctatagccgtcatcctgcggaaaatggaagagcaacttcctgaac P K I Q V Y S R H P A E N G K S N F L N tgctatgtttcggggtttcatccgtcggacattgaggtagacctgctgaagaacggtgaa C Y V S G F H P S D I E V D L L K N G E | | | | | | | |
| cgcattgagaaagtggagcacagcgatctcagcttcagtaaagattggtcctttacctg R  I  E  K  V  E  H  S  D  L  S  F  S  K  D  W  S  F  Y  L ttgtactacacggaatttacgcccacagagaaagacgaatatgcgtgtcgcgtgaatcac L  Y  Y  T  E  F  T  P  T  E  K  D  E  Y  A  C  R  V  N  H gtaaccctttcccagccgaaaatcgtcaaatgggatcgcgatatgggcggtggaggatca V  T  L  S  Q  P  K  I  V  K  W  D  R  D  M  G  G  G  G  S ggtggcatggatgaaaagaccaccggttggcgtggcggtcatgtggttgaaggcttagct G  G  M  D  E  K  T  T  G  W  R  G  G  H  V  V  E  G  L  A ggcgaactggaacagttgcgtgcacgtctggaacatcacccacaaggccaacgcgaaccg G  E  L  E  Q  L  R  A  R  L  E  H  H  P  Q  G  Q  R  E  P taactaactaacctgcaggcgatccggtaagatccggctgctaacaaagcccgaaaggaa -  L  T  N  L  Q  A  I  R  -  D  P  A  A  N  K  A  R  K  E gctgagttggctgctgccaccgctgagcaataactagcataaccccttggggcctctaaa A  E  L  A  A  A  T  A  E  Q  -  L  A  -  P  L  G  A  S  K cgggtcttgaggggttttttgctgaaaggaggaactatatccgga R  V  L  R  G  F  L  L  K  G  G  T  I  S  G Total size = 767 bps | | | | | | | |

Base pairs 1-101 are the T7 promoter and associated sequences.
Base pairs 102-122 encode the fixed peptide BZLF1.
Base pairs 123-488 encode a flexible linker-B2M-linker.
Base pairs 489-602 encode a streptavidin binding protein.
Base pairs 603-767 are a T7 termination sequence and associated sequences.

A further DNA template using a randomised epitope was also designed. In this template a His-tag was used for protein coupling to the beads. The DNA template was as follows:

| **T7 promoter** | **Random epitope 9 a.a.** | **Flexible linker** | **Human β2m** | **linker** | **HA tag** | **linker** | **T7 terminator** |
|---|---|---|---|---|---|---|---|
| ccatgggatctcgatcccgcgaaattaatacgactcactatagggagaccacaacggtttcc M G S R S R E I N T T H Y R E T T T V S ctctagaaataattttgtttaactttaagaaggagatataccatgnnknnknnknnknnk L - K - F C L T L R R Y T M X X X X X nnknnknnknnkggcggtggtggtggttctggcggcagtggcggtcaggcggcatt X X X X G G G G G S G G S G G S G G I caacgtaccccgaaaatccaggtgtatagccgtcatccagctgaaaacggcaaaagcaac Q R T P K I Q V Y S R H P A E N G K S N tttctgaactgctatgtaagcgggtttcatccttcggacattgaagtcgatctgctgaag F L N C Y V S G F H P S D I E V D L L K aatgggaacgcattgagaaagtggaacacagcgatttgtccttctcgaaagactggtcc N G E R I E K V E H S D L S F S K D W S ttctaccttctgtactacacggaatttactcccacagagaaagacgaatatgcatgtcgc F Y L L Y Y T E F T P T E K D E Y A C R gttaatcacgtcaccctcagtcagccgaaaatcgtgaaatgggatcgggatatgggtggc V N H V T L S Q P K I V K W D R D M G G ggcggatctggtggatacccgtatgatgttccggattatgcgtaactaactaacctgcag G G S G G Y P Y D V P D Y A - L T N L Q gcgatccggtaagatccggctgctaacaaagcccgaaaggaagctgagttggctgctgcc A I R - D P A A N K A R K E A E L A A A accgctgagcaataactagcataaccccttggggcctctaaacgggtcttgaggggtttt T A E Q - L A - P L G A S K R V L R G F ttgctgaaaggaggaactatatccggacccggg L L K G G T I S G P G | | | | | | | |
| Total size = 695 bps | | | | | | | |

Base pairs 1-107 are a T7 promoter and associated sequences.
Base pairs 108-134 encode a random epitope.
Base pairs 135-497 encode a flexible linker-B2M-linker.
Base pairs 498-551 encode a HA tag and linker.
Base pairs 525-695 are a T7 termination sequence and associated sequences.

Primers
5'-bio-for 5'-ccatgggatctcgatcccgcgaaatt-3'
5'-bio-rev 5'-cccgggtccggatatagttcctcctt-3'
5'-PCR-for 5'-ccatgggatctcgatcccgcgaaatt-3'
5'-PCR-rev 5'-cccgggtccggatatagttcctcctt -3'

### Experimental Methods

### Creating bead-primers

Dynabead M270 streptavidin beads were washed three times by separating the beads using a magnet, removing the supernatant, re-suspending the beads in binding and washing buffer (10mM Tris-HCl, 1mM EDTA, 2M NaCl). The appropriate quantity of biotinylated primers (5'-bio-for only, 5'-bio-rev only, or both) was then added to the re-suspended beads and the beads and primer were incubated on a rotor at room temperature. The amount of primers added was determined by the binding capacity suggested by the bead manufacturer (the maximum binding capacity of 1 mg, or 6-7 x 10⁷ of Dynabead M270 streptavidin to single-stranded oligonucleotides is 200 pmol).

After binding the primers to the beads, the beads are washed three times with binding and washing buffer to remove any unbound primer oligos. The beads with bound primer were then re-suspended in water and stored at 4°C.

In an alternative embodiment dual biotin labeling is employed at the 5' end of the primer. Using double the biotin proves significantly stronger in resisting the high temperature cycle during PCR. In addition, Polyethylene Glycol (PEG) spacer may be introduced between the dual biotins and the 5' end of the primer. This improves the accessibility of the polymerase enzyme in synthesizing the DNA template near the 5' end.

### Emulsion PCR

The emulsion oil for each PCR reaction was prepared in a universal tube as follows: 475 ul mineral oil, 22.5 ul Span-80, 2.5 ul Tween-80, 0.25 ul Triton X-100. Alternatively, ABIL EM 90 surfactant (Bis-PEG/PPG-14/14 Dimethicone, Cyclopentasiloxane, available from Degussa) may be used, this is more durable in PCR reactions. To equilibrate the emulsion oil, a magnetic stirrer was added to the tube. The tube was then placed on a magnetic spin at ≥1000 rpm in a cold room for 10 min.

Each aqueous PCR reaction (50 ul) was prepared as follows: approximately 10⁶ primer-coupled beads, 200 nM complement unmodified primers (vary according to the type of primers attached to the beads), 50nM unmodified forward primer, 200 uM PCR grade dNTPs, 1.5 mM MgCl, approximately 50 ng DNA template, and 2.5 unit Expand High Fidelity Enzyme Blend. The water-in-oil emulsion was prepared by slow addition of 50 ul aqueous PCR mixture into the spinning emulsion oil, the mix was left to spin for an additional 10 min. The emulsions were then aliquoted into 100 ul each, and subjected to the following PCR cycles: 94°C 2 min, 40 cycles of 94°C 30s, 61°C 30s, 72°C 1 min, then 72°C 7 min, followed by 4°C incubation.

After the PCR cycles, the same reactions were pooled together in a new 1.5 ml eppendorf tube. The emulsion was broken by adding 1 ml H₂O-saturated diethyl ether and vortexing for 5s. Alternatively isobutanol may be used. The broken emulsions were then centrifuged at 13,000 x g for 5 min at room temperature to recover the beads. The upper solvent phase (sometimes with white aggregates) was discarded. The washing was repeated a further two times. To remove any leftover ether, the tubes were vacuum centrifuged for 5 min at room temperature. (Alternatively, the tubes were left open in the fume hood for 10 min). The beads were re-suspended in 10 ul ddH₂O.

### Emulsion in vitro transcription/translation

The emulsion oil was prepared as described in the emulsion PCR method. Each in vitro transcription/translation reaction (RTS 100 E. coli HY kit, Roche) was prepared as follows: 12 ul E. coli lysate, 10 ul reaction mix, 12 ul amino acids (no methionine), 1 ul methionine, 5 ul reconstitution buffer, H₂O up to 40 ul. 10 ul of the emulsion PCR re-suspended beads was added to 40 ul of in vitro transcription/translation mix to form a 50 ul reaction. The 50 ul reaction was mixed thoroughly by pipetting.

The 50 ul in vitro transcription/translation mixture was then added to the oil and a spinning emulsion formed. The reaction was incubated at 30°C for 4 hours on a heat block.

After 30 minutes the emulsion was broken as described with reference to emulsion PCR. The beads were recovered as described with reference to emulsion PCT and re-suspended in 50 ul ddH₂O.

### Testing amplified double-stranded DNA tethering to the beads after emulsion PCT and IVTT

An XbaI restriction site (TCTAGA) is found at the 57^{th} base of the BZLF1 construct, and at the 63^{th} base of the randomized epitope construct, this site was used to confirm that the double-stranded DNA was tethered to the bead.

10 ul of IVTT re-suspended beads were taken and the beads were separated from the supernatant with a magnet. The beads were then re-suspended in fresh ddH₂O. The following restriction digestion was prepared: 1 ul XbaI enzyme (20 units), 0.2 ul bovine serum albumin 100 ug/ml, 2 ul NEBuffer 4 10X (50 mM potassium acetate, 20 mM Tris-acetate, 10 mM magnesium acetate, 1 mM DTT, pH 7.9), 6.8 ul ddH₂O, and added to the newly re-suspended beads. The beads and restriction enzyme mix were incubated at 37°C overnight. The digest was examined on a 1% agarose gel - Figure 2.

In the experiment depicted in Figure 2 the BZLF1 construct was used as the DNA template for all samples. Therefore if dsDNA was amplified and attached to the beads, XbaI restriction digest would cleave and leave approximately 700 bp of unbound DNA fragments which can be examined by the gel. Bands at 700 bps could be observed in samples with biotinylated primer-beads and DNA templates, regardless of forward or reverse primers. However, only 5'-biotin-reverse primer-beads gave a clear result (well 4), i.e. only single band at 700 bps. This could be due to the presence of the beads near the restriction recognition site, which leads to inaccurate binding of the enzyme on DNA. The presence of the smears in the supernatants suggest that a substantial amount of bacterial DNA was present in the E coli lysate.

### Examining the integrity of translated proteins - Western Blotting

20 ul of the resuspended beads produced according to the above section entitled "Emulsion in vitro transcription/translation" was mixed with 20 ul of Lithium Dodecyl Sulfate (LDS) 4X, and 40 ul of ddH₂O. The reaction was incubated at 75°C on a heat block for 10 min. The samples were then loaded onto NuPAGE 12% Bis-Tris Midi gel, which was submerged in a gel tank filled with NuPAGE MES SDS running buffer. The gel was run at 140V for 1.5 hours then transferred onto Hybond nitrocellulose membrane (pre-soaked with NuPAGE transfer buffer) with semi-dry Western blot transfer system for 30 min at 50 mA.

After transfer, the nitrocellulose membranes were blocked with blocking buffer (3% milk, PBST) for ≥ 1hr on a shaker in the cold room.

The membranes were then stained with mouse monoclonal anti-human beta-2-microglobulin (anti-BBM1), 1:500 in PBST for 1hr on a shaker at room temperature. (For staining with hemagglutinin tag, rat monoclonal anti-hemagglutinin conjugated to HRP was used at 1:500 dilution). After the staining the membranes were washed for 5 min with PBST three times. Then the membranes were stained with secondary antibody goat anti-mouse polyclonal antibody conjugated to horseradish peroxidase, 1:1000 in PBST for 1 hr on a shaker, room temperature. The membranes were washed again and developed by ECL developing reagent, filmed on X-ray films.

In the results, shown in Figure 3, the EBV protein (BZLF1 positive control) was translated in bead-emulsion IVTT and stained for human beta-2-microglobulin. The estimated size of this protein is 18.38 kDa, which correlates to the position of the bands in sample 1,2 and 3. The extra bands on the top of the wells are due to aggregration and impaired migration of the bead-protein-DNA complexes.

In the results shown in Figure 4 the EBV protein (BZLF1 positive control) and the randomised epitope protein construct were translated in bead-emulsion and stained for the hemagglutinin tag. Since the BZLF1 positive control sequence did not code the hemagglutinin sequence, the staining results were negative (sample 1 and 2). The estimated size of the randomised epitope protein was 15 kDa, which correlated to the position of the bands at sample 3 and 4.

### MHC class I refolding of bead-DNA-protein complexes

The experiments described above demonstrate that emulsion PCR, followed by emulsion disruption, followed by emulsion formation, followed by emulsion IVTT is achievable on a bead. The successful translation of human beta-2-microglobulin suggests that the 9 amino-acid random epitope can be translated successfully as well.

In order to rapidly screen the epitopes which bind to known MHC heavy chains, a refolding reaction was carried out. The correctly refolded bead-MHC-peptide complex was screened by ELISA.

### Refolding of the peptide, the β2 microglobulin and the HLA

To prepare the refolding mixture the bead-proteins were collected from the bead-emulsion IVTT reaction, with a typical volume of 50 ul. The refolding buffer was prepared as follows: for 1 L refolding buffer, 100 ml 1M Tris pH8,4 ml 0.5M EDTA, 84.28 g L-arginine HCl, 1.54 g reduced glutathione, 0.31 g oxidised glutathione. The buffer is equilibrated with a magnetic stirrer.

1 ml of refolding buffer was placed in a 1.5 ml eppendorf tube in the cold room, the tubes were fixed on a rotor. 50 ul of IVTT product was added to the 1 ml of refolding buffer. 10 ul of HLA heavy chain (stock 30 mg/ml) was slowly added into refolding buffer. For BZLF1 positive control, HLA-B8 is added (recombinant HLA-B*0801 synthesized in house). The refolding mixture and bead-protein and HLA was then left on the rotor overnight in the cold room. To reduce the amount of non-specific aggregates, the refolding mixture is centrifuged at 3000 rpm, for 5 min. The supernatant is then removed and concentrated by passing through a 15 ml centrifuge filter unit (Millipore). The tubes are spun at 1500 rpm for 5 min, to concentrate the volume to approximately 100 ul. The concentrate can then be stored at 4oC. The refolding mixture was then centrifuged at 4000rpm for 20 min. The supernatant was removed, to remove aggregates. A millipore 15ml concentrator was then used to concentrate the 1ml refolding mixture down to approximate 200 ul. 100 ul of the sample was loaded onto an ELISA well precoated with W6/32 - an antibody that will only recognise correctly refolded HLA and β2 microglobulin (mouse monoclonal anti-HLA-A/B/C from eBiosciences),1:300, and blocked with 3% BSA. The plate was incubated for 1 hr at 37°C. The plate was then washed and the wells loaded with rat monoclonal anti-b2m (1:1000). The plates were then incubated for 2 hours at RT. The plates were then washed with excess PBS by gentle aspiration in the wells, before adding 100 ul goat anti-rat polyclonal antibody conjugated to horseradish peroxidase (Abcam) at 1:1000 dilution. The plates were incubated for 1 hr at room temperature and washed again. 100 ul Tetramethyl Benzidine chromogen (TMB) solution (Invitrogen) was added to each well, and 5-10 min was waited for the colour change. 100 ul ELISA stop solution (Invitrogen) was then added to each well. Absorbance readings for each well at 450 nm was then collected by ELISA plate reader.

The results in Figure 5 demonstrate that the peptide, HLA and β2 microglobulin are correctly folded on the bead.

### Identification of epitopes for T-cell receptors and/or NK-cell receptors.

Beads produced as described above by emulsion PCR and IVTT displaying a peptide, known or random, the encoding DNA, β2 microglobulin and MHC or MHC-like molecule on their surface are applied to T-cells and/or NK-cells in suspension or attached to a support. The beads and cells are incubated for about 20 minutes at 37°C. Unbound beads are washed off by using size exclusion filtration or alternative approaches including differential magnetic migration of unbound beads compared to beads attached to cells. The DNA is then purified from the cells and beads. The recovered DNA is then amplified by PCR using primers designed to amplify the DNA encoding the peptide epitope. The amplified DNA is then sequenced. As the sequences are detected by PCR and sequencing, only a few cells are required to test the specificity. Visualisation of reactive cells using techniques such as flow cytometry is an alternative but requires larger numbers of cells.

The T-cells or NK-cells used in the method of the invention may comprise part of the cell mix in a homogenised tissue sample, purification of the T-cells or NK-cells from the sample is not necessary. The method allows a very small number of cells to be used, this is important if there is only a limited sample, such as a biopsy sample. Alternatively, the T-cells or NK-cells may be in a blood sample, for example, a blood sample from an individual infected with a pathogen such as staphylococcus, dengue or influenza. By using tissue samples from individuals with a known condition or infection the method of the invention will allow more to be learnt about disease pathogenesis and will also allow potential drug targets or biomarkers to be identified.

The peptide library used could be random or could be tailored for a particular application. For example, if looking a dengue virus infection, the peptide library could be based on known dengue proteins.

### Identification of peptides from a bead-protein-DNA library which recognise HLA-A*0201-restricted GILGFVFTL-influenza matrix specific T-cell clones

A2-specific T-cell clones (HLA-A*0201-restricted GILGFVFTL-influenza matrix specific T-cell clones) were washed with RPMI 1640 medium and re-suspended at 10⁴ cells in 400 ul RPMI 1640 medium for each reaction. 50 ul of a protein-DNA-bead resuspension was incubated with 10⁴ T-cell clones for 30 min at 37°C. The protein-DNA-beads used were as described earlier and comprised beads with DNA encoding a random peptide and beta-2-microglobulin attached thereto, which has been transcribed and translated in vitro on the beads to express the peptide and the beta-2-microglobulin which are anchored to the bead, HLA-A2 heavy chain, which is required to correctly present the peptide, was added exogenously. The peptide library size was in the order of 10exp7.

Each reaction was then transferred to a well of a 24-well Millipore Millicell filter culture plate, and the plate was spun at 1500 rpm, for 90 seconds. Unbound beads were washed through the filter, and the remaining cells were resuspended in ddH₂O and the reaction was transferred onto ice. Depending on the rate of reaction, if a large number of beads were internalized by the cells due to T-cell receptor-MHC complex interaction, cell lysis treatment was required to harvest the beads. If the beads were attached on the cell surface, and not internalised, the DNA could be amplified directly.

Standard PCR amplification (94°C 30 sec, 50°C 30 sec, 72°C 30 sec, 35 cycles) with primers flanking the randomized protein region was used. PCR samples were purified using the PCR purification kit (Qiagen). The purified DNA samples were then amplified by extension PCR with a 5' primer adding an additional 4 bases (CACC) on the 5' end of the DNA template. This was needed for directional TOPO cloning. The PCR samples were purified again with the PCR purification kit (Qiagen). A directional TOPO cloning reaction (pENTR Directional TOPO Cloning Kits, Invitrogen) was performed as described by the manufacturer's instructions. The TOPO cloned reactions were plated on kanamycin supplemented agar plates, and incubated at 37°C overnight The next day 20-50 colonies were picked and expanded by incubating the bacteria in 5 ml LB culture supplemented with kanamycin at 37°C with shaking until the OD600 is 0.5 or above. Plasmids were then extracted using the Qiagen Miniprep Kit according to the manufacturer's instructions. The recovered plasmids were then sequenced to understand the exact protein sequence which had interacted with the A2-specific T-cell clones.

The results of the sequencing showed that nine sequences were recovered. In this example conventional sequencing was used, but if high throughput sequencing was used many millions of sequences could be examined.

The sequences obtained using conventional cloning and sequencing in this example were:
1. Q V xxxxxx R
2. A I xxxxxx I
3. M A xxxxxx W
4. L A xxxxxxx
5. Q R xxxxxx A
6. M A xxxxxxxx R
7. S S xxxxxx V
8. G I xxxxxx L
9. C L xxxxxx D

Sequence 8 shows identical anchor residues to the influenza matrix peptide GILGFVFTL. Therefore 1/9 of the sequences potentially identified the epitope recognised by the specific T cells after just a single round of selection. Using large numbers of sequences generated with high throughput sequencing approaches, it will be possible to use bioinformatics to identify relevant sequences, for example through comparing sequences obtained from control cells. Alternative strategies may be to use varying times and temperatures of incubation of the beads with the cells and to use blocking strategies such as control beads (with known defined sequences) and/or using anti-CD8 to limit CD8/bead association. In addition it is possible to re-derive the beads using the sequences obtained from round 1 of selection in order to undertake further rounds of selection to identify relevant sequences.

## Claims

1. A method to identify a peptide and/or its encoding DNA, wherein the peptide binds to a T-cell receptor and/or an NK-cell receptor and/or an NKT-cell receptor, the method comprising: providing a carrier to which is attached a peptide and its encoding DNA; providing an MHC and/or an MHC-like molecule; and exposing the peptide to a T-cell receptor and/or an NK-cell receptor and/or an NKT-cell receptor in the presence of the MHC or MHC-like molecule wherein an MHC-like molecule has an MHC-like 3D structure and is able to present a peptide to a T-cell, NK-cell or NKT-cell.

2. The method of claim 1 further comprising providing β2 microglobulin together with the MHC and/or the MHC-like molecule, and exposing the peptide to a receptor in the presence of β2 microglobulin and the MHC or MHC-like molecule.

3. The method of any of claim 1 or 2, wherein the peptide, the MHC or MHC-like molecule is configured to present the peptide to a T-cell receptor and/or an NK-cell receptor and/or an NKT-cell receptor.

4. The method of claim 2, wherein the peptide, the MHC or MHC-like molecule and/or the β2 microglobulin is configured to present the peptide to a T-cell receptor and/or an NK-cell receptor and/or an NKT-cell receptor.

5. The method of any preceding claim wherein the method is performed *in vitro,* and/or wherein the MHC or MHC-like molecule is the full length naturally occurring MHC or MHC-like molecule, or wherein the MHC or MHC-like molecule is a functional variant of an MHC or MHC-like molecule, and/or wherein the carrier is multivalent and/or wherein the peptide used is between 4 and 20 amino acids.

6. The method of any preceding claim wherein the method further comprises the step of recovering the peptide, and/or its encoding DNA, from a carrier which bound to a T-cell receptor and/or an NK-cell receptor and/or an NKT-cell receptor and optionally wherein the recovered peptide or DNA is sequenced.

7. The method of any preceding claim wherein the β2 microglobulin and/or the MHC or MHC-like molecule is added exogenously to the peptide, or wherein the β2 microglobulin and/or the MHC or MHC-like molecule is provided connected to the peptide via a linker.

8. The method of any preceding claim wherein the carrier is a solid support and optionally wherein the solid support is a bead.

9. The method of any preceding claim wherein the DNA and/or the protein are generated by *in vitro* PCR and/or *in vitro* transcription/translation.

10. A method to identify a DNA encoding a peptide which binds to a T-cell receptor and/or an NK-cell receptor and/or an NKT-cell receptor, the method comprising providing a bead carrier to which is attached a peptide and the DNA encoding the peptide, exposing the peptide in the presence of β2 microglobulin and an MHC or MHC-like molecule to a T-cell receptor and/or an NK-cell receptor and/or an NKT-cell receptor, recovering any beads that bound to or were internalised by the T-cell receptor and/or the NK-cell receptor and/or the NKT-cell receptor, wherein an MHC-like molecule has an MHC-like 3D structure and is able to present a peptide to a T-cell, NK-cell or NKT-cell, and optionally comprising sequencing the DNA on the recovered beads, and optionally wherein the peptide and β2 microglobulin are provided connected by a flexible linker, such that both are attached to the bead.

11. The method of any of the preceding claims wherein the T cells and/or NK cells and/or NKT cells expressing the T-cell receptor or NK-cell receptor or NKT-cell receptor are provided in a tissue sample.

12. A carrier to which is attached a peptide, DNA encoding the peptide, and β2 microglobulin and/or an MHC or MHC-like molecule, wherein an MHC-like molecule has an MHC-like 3D structure and is able to present a peptide to a T-cell, NK-cell or NKT-cell.

13. The carrier of claim 12 wherein the β2 microglobulin and/or the MHC or MHC-like molecule are attached to the carrier as part of a larger construct which includes the peptide.

14. The use of a carrier according to claim 12 or 13 to identify an epitope recognised by a T-cell receptor and/or an NK-cell receptor and/or an NKT-cell receptor.

15. A kit for screening for a T-cell epitope and/or an NK-cell epitope and/or an NKT-cell epitope, wherein the kit comprises a carrier to which is attached a peptide, DNA encoding the peptide, and β2 microglobulin and/or an MHC or MHC-like molecule, wherein an MHC-like molecule has an MHC-like 3D structure and is able to present a peptide to a T-cell, NK-cell or NKT-cell, and optionally further including instructions to fold the peptide, β2 microglobulin and/or MHC or MHC-like molecule such that the peptide is in the correct configuration to be recognised by a T-cell receptor and/or an NK-cell receptor and/or an NKT-cell receptor, and optionally further including instructions to expose the carrier to T-cell receptors and/or NK-cell receptors and/or NKT-cell receptors and to isolate peptides, or the DNA encoding the peptides, that bind to the T-cell receptors and/or NK-cell receptors and/or NKT-cell receptors.

## Patentansprüche

1. Verfahren zur Identifizierung eines Peptids und/oder seiner kodierenden DNA, wobei sich das Peptid an einen T-Zellen-Rezeptor und/oder einen NK-Zellen-Rezeptor und/oder einen NKT-Zellen-Rezeptor bindet, wobei das Verfahren Folgendes umfasst: Bereitstellen eines Trägers, mit dem ein Peptid und seine kodierende DNA gebunden sind; Bereitstellen eines MHC- und/oder MHC-artigen Moleküls; und Aussetzen des Peptids an einen T-Zellen-Rezeptor und/oder einen NK-Zellen-Rezeptor und/oder einen NKT-Zellen-Rezeptor in Gegenwart des MHC- oder MHC-artigen Moleküls, wobei ein HC-artiges Molekül eine MHC-artige, 3-dimensionale Struktur aufweist und ein Peptid einer T-Zelle, einer NK-Zelle oder einer NKT-Zelle präsentieren kann.

2. Das Verfahren entsprechend Anspruch 1, das weiterhin Bereitstellen von β2-Mikroglobulin zusammen mit dem MHC- und/oder dem MHC-artigen Moleküls und Aussetzen des Peptids an einen Rezeptor in Gegenwart des β2-Mikroglobulins und des MHC- oder MHC-artigen Moleküls umfasst.

3. Das Verfahren entsprechend Anspruch 1 oder 2, wobei das Peptid, das MHC- oder MHC-artige Molekül so konfiguriert ist, dass es das Peptid einem T-Zellen-Rezeptor und/oder einem NK-Zellen-Rezeptor und/oder einem NKT-Zellen-Rezeptor präsentiert.

4. Das Verfahren entsprechend Anspruch 2, wobei das Peptid, das MHC- oder MHC-artige Molekül und/oder das β2-Mikroglobulin so konfiguriert ist, dass es das Peptid einem T-Zellen-Rezeptor und/oder einem NK-Zellen-Rezeptor und/oder einem NKT-Zellen-Rezeptor präsentiert.

5. Das Verfahren entsprechend einem der vorhergehenden Ansprüche, wobei das Verfahren *in vitro* durchgeführt wird und/oder wobei das MHC- oder MHC-artige Molekül das natürlich vorkommende MHC- oder MHC-artige Molekül in voller Länge ist oder wobei das MHC- oder MHC-artige Molekül eine funktionale Variante eines MHC- oder MHC-artigen Moleküls ist und/oder wobei der Träger multivalent ist und/oder wobei das Peptid zwischen 4 und 20 Aminosäuren verwendet wird.

6. Das Verfahren entsprechend einem der vorhergehenden Ansprüche, wobei das Verfahren zudem den Schritt der Wiedergewinnung des Peptids und/oder seiner kodierenden DNA von einem Träger umfasst, das/die an einen T-Zellen-Rezeptor und/oder einen NK-Zellen-Rezeptor und oder einen NKT-Zellen-Rezeptor gebunden ist und optional wobei das wiedergewonnen Peptid oder DNA sequenziert ist.

7. Das Verfahren entsprechend einem der vorhergehenden Ansprüche, wobei das β2-Mikroglobulin und/oder das MHC- und das MHC-artige Molekül dem Peptid exogen zugefügt wird oder wobei das β2-Mikroglobulin und/oder das MHC- und das MHC-artige Molekül an das Peptid über einen Binder verbunden bereitgestellt wird.

8. Das Verfahren entsprechend einem der vorhergehenden Ansprüche, wobei der Träger eine solide Stütze ist und optional wobei die solide Stütze ein Kügelchen ist.

9. Das Verfahren entsprechend einem der vorhergehenden Ansprüche, wobei die DNA und/oder das Protein durch *In-Vitro*-PCR und/oder *In-Vitro*-Transkription/Translation erzeugt wird.

10. Ein Verfahren zu Identifizierung einer DNA, die ein Peptid kodiert, das sich an einen T-Zellen-Rezeptor und/oder einen NK-Zellen-Rezeptor und/oder NKT-Zellen-Rezeptor bindet, wobei das Verfahren das Bereitstellen eines Kügelchenträgers umfasst, an den ein Peptid und die das Peptid kodierende DNA angehängt sind, Aussetzen des Peptids in Gegenwart von β2-Mikroglobulin und eines MHC- oder MHC-artigen Moleküls an einen T-Zellen-Rezeptor und/oder einen NK-Zellen-Rezeptor und/oder einen NKT-Zellen-Rezeptor, Wiedergewinnung jeglicher Kügelchen, die an den T-Zellen-Rezeptor und/oder den NK-Zellen-Rezeptor und(der NKT-Zellenrezeptor gebunden waren oder von diesem internalisiert wurden, wobei ein MHC-artiges Molekül eine MHC-artige, 3-dimensionale Struktur aufweist und ein Peptid einer T-Zelle, einer NK-Zelle oder einer NKT-Zelle präsentieren kann, und optional das Sequenzieren der DNA an den wiedergewonnenen Kügelchen umfasst und optional wobei das Peptid und das β2-Mikroglobulin durch einen flexiblen Binder verbunden bereitgestellt werden, so dass beide am Kügelchen befestigt sind.

11. Das Verfahren entsprechend einem der vorhergehenden Ansprüche, wobei die T-Zellen und/oder NK-Zellen und/oder NKT-Zellen, die den T-Zellen-Rezeptor oder NK-Zellen-Rezeptor oder NKT-Zellen-Rezeptor exprimieren, in einer Gewebsprobe bereitgestellt werden.

12. Ein Träger, an dem ein Peptid, eine das Peptid kodierende DNA und β2-Mikroglobulin und/oder ein MHC- oder MHC-artiges Molekül gebunden sind, wobei ein MHC-artiges Molekül eine MHC-artige, 3-dimensionale Struktur aufweist und ein Peptid einer T-Zelle, einer NK-Zelle oder einer NKT-Zelle präsentieren kann.

13. Der Träger von Anspruch 12, wobei das β2-Mikroglobulin und/oder das MHC- oder MHC-artige Molekül am Träger als Teil eines größeren Konstrukts angebracht sind, zu dem das Peptid gehört.

14. Die Verwendung eines Trägers entsprechend Anspruch 12 oder 13 zur Identifizierung eines Epitops, das von einem T-Zellen-Rezeptor und/oder NK-Zellen-Rezeptor und/oder NKT-Zellen-Rezeptor erkannt wird.

15. Ein Kit zur Untersuchung auf ein T-Zellen-Epitop und/oder ein NK-Zellen-Epitop und/oder ein NKT-Zellen-Epitop, wobei das Kit einen Träger umfasst, an den ein Peptid, das Peptid kodierende DNA und β2-Mikroglobulin und/oder ein MHC- oder MHC-artiges Molekül angehängt sind, wobei ein MHC-artiges Molekül eine MHC-artige, 3-dimensionale Struktur aufweist und ein Peptid einer T-Zelle, einer NK-Zelle oder einer NKT-Zelle präsentieren kann, und optional weiterhin Anweisungen zum Falten des Peptids, β2-Mikroglobulins und/oder MHC- oder MHC-artigen Moleküls, so dass das Peptid in der korrekten Konfiguration ist, um von einem T-Zellen-Rezeptor und/oder NK-Zellen-Rezeptor und/oder NKT-Zellen-Rezeptor erkannt zu werden, und optional zudem Anweisungen enthält, den Träger T-Zellen-Rezeptoren und/oder NK-Zellen-Rezeptoren und/oder NKT-Zellen-Rezeptoren auszusetzen und Peptide oder die Peptipe kodierende DNA zu isolieren, die sich an die T-Zellen-Rezeptoren und/oder NK-Zellen-Rezeptoren und/oder NKT-Zellen-Rezeptoren binden.

## Revendications

1. Un procédé d'identification d'un peptide et/ou de son ADN de codage, où le peptide se lie à un récepteur de cellules T et/ou un récepteur de cellules NK et/ou un récepteur de cellules NKT, le procédé comprenant : la fourniture d'un porteur auquel est attaché un peptide et son ADN de codage, la fourniture d'une molécule MHC et/ou de type MHC, et l'exposition du peptide à un récepteur de cellules T et/ou un récepteur de cellules NK et/ou un récepteur de cellules NKT en présence de la molécule MHC ou de type MHC où une molécule de type MHC possède une structure 3D de type MHC et est capable de présenter un peptide à une cellule T, une cellule NK ou une cellule NKT.

2. Le procédé selon la Revendication 1 comprenant en outre la fourniture d'une β2-microglobuline conjointement avec la molécule MHC et/ou de type MHC et l'exposition du peptide à un récepteur en présence de la β2-microglobuline et de la molécule MHC ou de type MHC.

3. Le procédé selon l'une quelconque des Revendications 1 ou 2, où le peptide, la molécule MHC ou de type MHC sont configurés de façon à présenter le peptide à un récepteur de cellules T et/ou un récepteur de cellules NK et/ou un récepteur de cellules NKT.

4. Le procédé selon la Revendication 2, où le peptide, la molécule MHC ou de type MHC et/ou la β2-microglobuline sont configurés de façon à présenter le peptide à un récepteur de cellules T et/ou un récepteur de cellules NK et/ou un récepteur de cellules NKT.

5. Le procédé selon l'une quelconque des Revendications précédentes où le procédé est exécuté *in vitro,* et/ou où la molécule MHC ou de type MHC est la molécule MHC ou de type MHC de longueur complète existant à l'état naturel, ou où la molécule MHC ou de type MHC est une variante fonctionnelle d'une molécule MHC ou de type MHC, et/ou où le porteur est polyvalent et/ou où le peptide utilisé est entre 4 et 20 acides aminés.

6. Le procédé selon l'une quelconque des Revendications précédentes où le procédé comprend en outre l'opération de récupération du peptide, et/ou de son ADN de codage, à partir d'un porteur qui s'est lié à un récepteur de cellules T et/ou un récepteur de cellules NK et/ou un récepteur de cellules NKT, et éventuellement où le peptide ou l'ADN récupéré est séquencé.

7. Le procédé selon l'une quelconque des Revendications précédentes où la β2-microglobuline et/ou la molécule MHC ou de type MHC sont ajoutées de manière exogène au peptide, ou où la β2-microglobuline et/ou la molécule MHC ou de type MHC sont fournies rattachées au peptide par l'intermédiaire d'un lieur.

8. Le procédé selon l'une quelconque des Revendications précédentes où le porteur est un support solide et éventuellement où le support solide est une bille.

9. Le procédé selon l'une quelconque des Revendications précédentes où l'ADN et/ou la protéine sont générés par PCR *in vitro* et/ou transcription/traduction *in vitro.*

10. Un procédé d'identification d'un ADN codant un peptide qui se lie à un récepteur de cellules T et/ou un récepteur de cellules NK et/ou un récepteur de cellules NKT, le procédé comprenant la fourniture d'un support à billes auquel est attaché un peptide et l'ADN codant le peptide, l'exposition du peptide en présence d'une β2-microglobuline et d'une molécule MHC ou de type MHC à un récepteur de cellules T et/ou un récepteur de cellules NK et/ou un récepteur de cellules NKT, la récupération de toutes les billes qui se sont liées à ou ont été internalisées par le récepteur de cellules T et/ou le récepteur de cellules NK et/ou le récepteur de cellules NKT, où une molécule de type MHC possède une structure 3D de type MHC et est capable de présenter un peptide à une cellule T, une cellule NK ou une cellule NKT, et éventuellement comprenant le séquençage de l'ADN sur les billes récupérées, et éventuellement où le peptide et la β2-microglobuline sont fournis rattachés par un lieur flexible de sorte que les deux soient rattachés à la bille.

11. Le procédé selon l'une quelconque des Revendications précédentes où les cellules T et/ou les cellules NK et/ou les cellules NKT exprimant le récepteur de cellules T ou le récepteur de cellules NK ou le récepteur de cellules NKT sont fournies dans un échantillon de tissu.

12. Un porteur auquel est attaché un peptide, un ADN codant le peptide et une β2-microglobuline et/ou une molécule MHC ou de type MHC, où une molécule de type MHC possède une structure 3D de type MHC et est capable de présenter un peptide à une cellule T, une cellule NK ou une cellule NKT.

13. Le porteur selon la Revendication 12 où la β2-microglobuline et/ou la molécule MHC ou de type MHC sont attachées au porteur dans le cadre d'une construction plus grande qui comprend le peptide.

14. L'utilisation d'un porteur selon la Revendication 12 ou 13 destiné à l'identification d'un épitope reconnu par un récepteur de cellules T et/ou un récepteur de cellules NK et/ou un récepteur de cellules NKT.

15. Un kit de criblage pour un épitope de cellule T et/ou un épitope de cellule NK et/ou un épitope de cellule NKT, où le kit contient un porteur auquel est attaché un peptide, un ADN codant le peptide et une β2-microglobuline et/ou une molécule MHC ou de type MHC, où une molécule de type MHC possède une structure 3D de type MHC et est capable de présenter un peptide à une cellule T, une cellule NK ou une cellule NKT, et éventuellement en outre comprenant des instructions destinées à plier le peptide, la β2-microglobuline et/ou la molécule MHC ou de type MHC de sorte que le peptide soit dans la configuration correcte pour être reconnu par un récepteur de cellules T et/ou un récepteur de cellules NK et/ou un récepteur de cellules NKT, et éventuellement en outre comprenant des instructions destinées à exposer le porteur à des récepteurs de cellules T et/ou à des récepteurs de cellules NK et/ou à des récepteurs de cellules NKT et à isoler des peptides, ou l'ADN codant les peptides, qui se lient aux récepteurs de cellules T et/ou aux récepteurs de cellules NK et/ou aux récepteurs de cellules NKT.
